# EUROPEAN PATENT SPECIFICATION

(11) **EP 3 052 165 B1**
(45) Date of publication and mention of the grant of the patent: **20.03.2019**
(21) Application number: 14789443.0
(22) Date of filing: 30.09.2014
(51) Int. Cl.: A61M 5/32, A61M 5/142, A61M 5/20

(54) **NEEDLE RETRACTION MECHANISM FOR AUTOINJECTOR**
NADELRÜCKZUGSMECHANISMUS FÜR EINEN AUTOINJEKTOR
MÉCANISME DE RETRACTION D'AIGUILLE POUR INJECTEUR AUTOMATIQUE

(30) Priority: 30.09.2013 US 201361884597 P; 04.12.2013 US 201314096977; 21.02.2014 US 201414186403; 30.06.2014 US 201462019066 P
(43) Date of publication of application: 10.08.2016
(73) Proprietor: Medimop Medical Projects Ltd., 43665 Ra'anana (IL)
(72) Inventor: CABIRI, Oz, 71799 Macabim-Reut (IL); HEZKIAHU, Ran, 4668346 Herzliya (IL)
(74) Representative: Lippert Stachow Patentanwälte Rechtsanwälte
(86) International application number: PCT/US2014/058446
(87) International publication number: WO 2015/048799

(56) References cited:
- US-A1- 2009 093 792

## Description

### BACKGROUND OF THE INVENTION

The present invention, in some embodiments thereof, relates to a needle point safeguarding mechanism and, more particularly, but not exclusively, to a retraction mechanism of an autoinjector.

U.S. Patent 7530964 to Cabiri discloses a needle device having a needle retraction mechanism that retracts the needle upon removing the device from the skin surface (either intentionally or unintentionally). Once the needle is retracted, the device is rendered inoperative. The needle can be further made inoperative by bending it when one attempts to reuse the device. In another embodiment, a needle opening formed in the base of the housing can be covered to render the needle inoperative when one attempts to reuse the device. In another embodiment, the needle device instead has a needle shield that automatically covers the needle after use.

U.S. Patent No. 8,348,898 to Cabiri, discloses a needle assembly adapted for fluid communication with a vial containing a substance to be delivered to a subject, the needle assembly including a needle held in a needle holder, the needle holder confined to move in a housing, and an activation mechanism for activating delivery of the substance through the needle, the activation mechanism including a safety latch that initially impedes movement of the needle holder, wherein when the safety latch is placed on the subject, the safety latch moves to a position that permits moving the needle holder to cause the needle to protrude outwards of the housing to pierce the subject to allow administration of the substance to the subject, characterized by a biasing device arranged to apply a biasing force on the needle to cause the needle to protrude outwards of the housing to pierce the subject, and needle release apparatus including a needle arrestor that initially blocks movement of the biasing device and which releases the biasing device when the safety latch moves to the position that permits moving the needle holder to cause the needle to protrude outwards of the housing.

U.S. Patent Application Publication No. 2009/093,792 to Gross and Cabiri discloses an apparatus for administering a substance to a subject. A vial contains the substance and a stopper is disposed within the vial and is slidably coupled to the vial. A first threaded element is (a) rotatable with respect to the vial and (b) substantially immobile proximally with respect to the vial during rotation of the first threaded element. A second threaded element is threadedly coupled to the first threaded element. At least a distal end of the second threaded element is substantially non-rotatable with respect to the vial, and the distal end of the second threaded element defines a coupling portion that couples the second threaded element to the stopper. The first threaded element, by rotating, linearly advances the stopper and at least the distal end of the second threaded element toward a distal end of the vial. Other embodiments are also described.

Additional background art includes International Patent Application Publication No. WO/2013/104414 to SANOFI-AVENTIS DEUTSCHLAND GMBH, U.S. Patent Application Publication No. 2011/0166509 to Gross and Cabiri, U.S. Patent Application Publication No. 2012/0130344 to Ebbett, U.S. Patent No. 8,267,890 to Alchas and U.S. Patent Application Publication No. 2009/0012494 to Yeshurun.

### SUMMARY OF THE INVENTION

According to the present invention there is provided a drug injector having a needle safeguard mechanism including a housing with a shielded location; a point of the needle protruding from the housing; a retractor mechanically coupled to the needle, the retractor retracting the point to the shielded location; and a sensor operationally linked to the retractor, the sensor sensing a resistance to discharging of the drug through the needle; wherein the retractor is responsive to the sensor to retract the point to the shielded location in response to the resistance. According to some embodiments of the invention, said retractor includes a support of the needle and the support reverts to a retracted state in response to the resistance.

According to some embodiments of the invention, the support includes an interference element, and the resistance overcomes the interference element.

According to some embodiments of the invention, the interference element includes an annular snap.

According to some embodiments of the invention, the support includes a telescoping assembly. According to some embodiments of the invention, the telescoping assembly is held in an extended mode by an interference element.

According to some embodiments of the invention, the drug injector further includes a driver driving the discharging and the driver is supported by the support, and in response to the resistance the driver produces a stress on the support and the stress reverts the support to the retracted state.

According to some embodiments of the invention, the drug injector further includes a plunger and the driver drives the discharging by imparting a linear motion to the plunger and the resistance is to the linear motion of the plunger.

According to some embodiments of the invention, the drug injector further includes a lock mechanically coupled to the needle, the lock retaining the point in the protruding position; and wherein the retractor is operationally linked to the lock to release the lock in response to the resistance.

According to some embodiments of the invention, the sensor is further sensitive to a second condition, and the lock retains the point in the protruding position until the second condition is met.

According to some embodiments of the invention, the point is biased to the shielded position. According to some embodiments of the invention, the lock includes at least one element selected from the group consisting of an interference element, an annular snap, a rib snap, a flange and a groove.

According to some embodiments of the invention, the second condition is a volume of the discharging passing a predetermined threshold.

According to some embodiments of the invention, the sensor senses a torque.

According to some embodiments of the invention, the retracting is in response to a change in the resistance.

According to some embodiments of the invention, the retracting is in response to an increase in the resistance.

According to some embodiments of the invention, the retracting is in response to a decrease in the resistance.

According to some embodiments of the invention, the retracting is in response to the resistance passing a threshold.

According to an aspect of some or any embodiments of the present invention there is provided a method of safeguarding a needle of an injector comprising: discharging a drug through the needle; sensing a resistance to the discharging, and safeguarding the needle in response to the resistance.

According to some or any embodiments of the invention, the safeguarding is in response to the resistance passing a predetermined threshold.

According to some or any embodiments of the invention, the safeguarding is in response to a change in the resistance.

According to some or any embodiments of the invention, the safeguarding is in response to an increase in the resistance.

According to some or any embodiments of the invention, the safeguarding is in response to a decrease in the resistance.

According to some or any embodiments of the invention, the method further includes locking the needle in an extended position during the discharging; and the safeguarding includes releasing the locking.

According to some or any embodiments of the invention, the resistance occurs upon at least one event selected from the group consisting of a completion of the discharging, a fluid leak, jamming of a mechanical component, and disconnection of a mechanical component and obstruction of a fluid flowpath.

According to some or any embodiments of the invention, the method further includes inhibiting the safeguarding until a second condition is met.

According to some or any embodiments of the invention, the second condition includes the discharging passing a predetermined volume.

According to some or any embodiments of the invention, the volume of discharging is measured by counting motor rotations.

According to some or any embodiments of the invention, the needle is a component of a pre filled syringe inside autoinjector and the method further includes: placing the autoinjector on a patient; extending the needle to an extended position subsequent to the placing and prior to the discharging, and the safeguarding includes retracting the needle to a retracted position.

According to some or any embodiments of the invention, the method further includes indicating that it is safe to remove the autoinjector after the retracting.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIG. 1 is a flowchart illustrating a method of safeguarding a needle of an autoinjector according to an embodiment of the present invention;
FIG. 2 is a illustrating a method of injecting a drug according to an embodiment of the present invention;
FIG. 3 is a state diagram of an autoinjector according to an embodiment of the present invention;
FIGS. 4A-D are schematic illustrations of a needle safeguarding mechanism that is triggered by a linear force resulting from resistance to discharge of a drug according to an exemplary embodiment of the present invention;
FIGS. 5A-C are schematic side view illustrations of an autoinjector with a needle safeguarding mechanism including concentric cylindrical telescoping elements according to an exemplary embodiment of the present invention;
FIGS. 6A-C are schematic side view illustrations of a lock of a needle safeguarding mechanism triggered by a torque according to an embodiment of the present invention;
FIGS. 7A,B illustrate a schematic side view and a top cross-sectional view respectively of a needle safeguarding mechanism triggered by a torque according to an embodiment of the present invention;
FIGS. 8A,B illustrate a schematic side view and a top cross-sectional view respectively of a needle safeguarding mechanism triggered by a torque and a second condition according to an embodiment of the present invention;
FIG. 9A-F are detailed schematic views illustrating states of an autoinjector according to an embodiment of the present invention;
FIGS. 10A-E illustrate a needle safeguarding mechanism triggered by a torque and a linear force according to an embodiment of the present invention;
Figs. 11A-C illustrate a manual trigger for a needle protector of an autoinjector according to an embodiment of the present invention;
Figs. 12A,B illustrate an optional non-circular cross section for syringe according to an embodiment of the present invention; and
Figs 13A-D illustrate an autoinjector with a motor switch located in the main body of the injector in accordance with some embodiments of the current invention.

### DESCRIPTION OF SPECIFIC EMBODIMENTS OF THE INVENTION

The present invention relates to a needle point safeguarding mechanism and, more particularly, to a retraction mechanism of an autoinjector.

### Overview

### 1 Needle protection mechanism

An aspect of some embodiments of the present invention relates to a needle safety mechanism for an autoinjector. Optionally, an unshielded needle may be safeguarded in response to resistance and/or a change of resistance to discharging of the injector payload.

In an exemplary embodiment, a lock may hold a needle in an unshielded position. The lock may optionally act as a support for a discharge driver. Increasing resistance to discharge may increase the stress on the driver. When the stress on the driver increases beyond a threshed and/or decreases beyond a threshold the lock may be released and/or the needle retracted to a safe location. For the sake of the current disclosure retraction of a needle may include pulling a needle point back into a shielded location without changing the length and/or shape of the shielding; and/or retracting may include extending the housing of the injector and/or a shield ahead of a needle point such that the needle is left in a shielded location.

In some embodiments, a support for a needle assembly may include a telescoping assembly. Optionally, the telescoping assembly may retract the needle (for example by contracting) in response to a stress from a driver.

In some embodiments, the safeguarding mechanism may include a sensor sensitive to a linear force from the driver. For example, a pushing force passes a threshold, a lock may be released moving the needle to the retracted configuration.

In some embodiments, the force to insert the needle to the skin of a patient may range for example between 0.1 to 0.5 N. Optionally, the force required to inject the drug (for example the force on a syringe plunger) may range for example between 5 to 20 N.

In some embodiments a needle protection mechanism may be triggered by a linear force greater than, for example, between 10 to 30 N.

For example, an autoinjector may be activated by manually pushing with enough force to insert the needle. The device may then apply an injection force to inject a drug. Once the entire drug is injected and/or when there is an obstruction and/or occlusion, the injection force may rise until it passes a threshold triggering safeguarding of the needle and/or ending injection.

For example in the event of an occlusion and/or at the end of delivery, the linear force generated by the device may increase to the level of up to 60 N. A needle safeguarding mechanism may include a sensor that is sensitive to the force. For example the sensor may include a snap that gives way at 40 N returning the needle to the retracted position.

In some embodiments, the stress to inject a medicine and/or to trigger safeguarding of a needle may include a torque. For example, injection of medicine may be driven by a plunger. The plunger may optionally be driven by a threaded assembly, for example a threaded screw and/or teeth and/or a telescoping assembly. Optionally the pitch of the teeth and/or an associated screw may range for example between 0.5 and 2 mm. The diameter of the screw may range for example between 3 and 12 mm. The torque to power injection may range for example between 0.2 and 1.0 N*cm. The trigger torque (the torque at which the needle safeguarding is triggered) may range for example between to 2-4 N*cm.

In some embodiments a safety mechanism may include linear movement of the ranging between 5 to 15 mm. For example movement of the safety mechanism may include extension of a needle during insertion and/or retraction of the needle and/or extensions of a safety shield and/or retraction of a safety shield. Optionally a needle insertion length (for example the length of needle inserted into a patient) may range for example between 5 to 10mm.

During injection, the linear movement of a plunger may range for example between 10-50mm. The length of movement of the plunger may vary for example with the volume of medicine to be injected that may range for example between 0.5 to 3ml.

In some embodiments, a sensor of a safeguarding mechanism may be sensitive to a torque. For example, the needle may be retracted when the mechanism is exposed to a twisting moment. Optionally, discharge may be driven by a torque. For example the driver may apply torque to threaded element pushing a plunger. When the torque on the driver reaches a threshold value, the needle may be released and/or retracted and/or a needle shield may be deployed.

In some embodiments an interference element (for example a snap) may provide resistance to retraction. For example, an annular ring may impede contraction of a telescoping assembly. Alternatively or additionally a rib may impede twisting of a support structure. When stress from the driver passes a threshold, the stress may optionally overcome the interference element. Overcoming the interference element may for example revert the support to a retracted configuration.

In some embodiments, a stress resulting from resistance to discharge may trigger deployment of a needle shield. The needle shield may optionally move to shield the needle in reaction to the increased stress.

### 2 Manual needle safety retraction mechanism

An aspect of some embodiments of the present invention relates to a manual retraction mechanism for an autoinjector. The manual retraction mechanism may allow a user to retract the needle of the autoinjector to a safe position in case of for example a malfunction of the injector. For example the safety retraction mechanism may include a release switch. When activated by the user, the release switch may permanently retract the needle into the injector. Alternatively or additionally the release switch may trigger deployment of a needle shield. For example, the casing of the injector may be biased to an extended configuration covering the needle. When the autoinjector is activated the casing may locked in a contracted configuration unshielding the needle. When the user pushes a switch, the lock may be permanently released, covering the needle and preventing reuse and/or a needle stick hazard.

### 3 Flexible cover to peel adhesive protector

An aspect of some embodiments of the present invention relates to a mechanism to peel an adhesive protector when a safety cover is pulled off of an autoinjector. For example the safety cover may be flexible and/or a hinged and/or may be anchored to an edge of the adhesive cover. As the safety cover is pulled away the pulling force may be transferred to a peeling force at the edge of the adhesive protector.

### 4 Stabilized manually held autoinjector

An aspect of some embodiments of the present invention relates to a manually held autoinjector with stabilization. For example, while in use the auto injector may be held to the skin of a patient by a user, for example the user may be a caretaker and/or the patient himself. In some cases, it may be difficult for a user to manually hold an autoinjector immobile enough and/or for long enough to complete injection. The auto injector may include an adhesive to increase stability of the injector. The adhesive may hold the activation zone of the injector and/or the injection zone and/or a needle aperture and/or a needle stable with respect to the skin of the patient.

In some embodiments, a syringe may be held substantially perpendicular to a surface contacting the user's skin. For example, the syringe may be held at an angle ranging between 75° to 105° to the user's skin during injection. For example, holding the injector immobile may include holding a needle at an injection depth of between 5 to 10 mm. For example, holding the injector immobile may include not sliding the injector along the patient's skin and/or straining the needle enough to bend and/or occlude the needle.

In some embodiments the injector may be held substantially immobile with respect to the skin of a patient for a time ranging for example between 10 to 600 seconds and/or the injector may be held substantially immobile with respect to the skin of a patient for a time ranging for example between 30 to 180 seconds. For example the injector may inject of volume of drug ranging between 0.5 to 3ml.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

### Exemplary embodiments

### 1 Method of safeguarding a needle

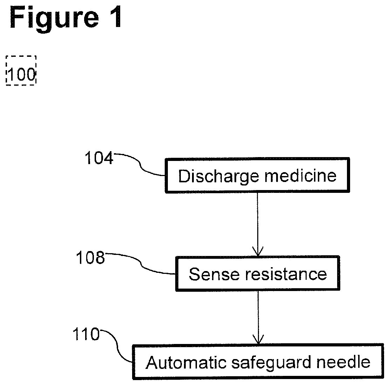

Referring now to the drawings, Fig. 1 illustrates a method of safeguarding a needle of an autoinjector according to an embodiment **100** of the present invention. In the exemplary method, when medicine is being discharged **104** from the autoinjector there may be a resistance to the discharging **104.** The resistance may be sensed **108.** When the resistance reaches a threshold level the needle may be safeguarded **110.**

In some embodiments of an autoinjector, the resistance to discharge may change at some point during the injection. For example, there may be an increase in resistance to movement of a plunger when it reaches the end of a syringe. For example, there may be an increase in resistance to movement of a plunger when a fluid path becomes obstructed. The change in resistance may be sensed **108** by, for example, a pressure sensitive trigger. Optionally increased pressure from the plunger may, trigger safeguarding **110** the needle. For example safeguarding 110 may include retracting the needle tip to a shielded location and/or movement of a shield to cover the needle tip.

### 2 Method of injecting a drug

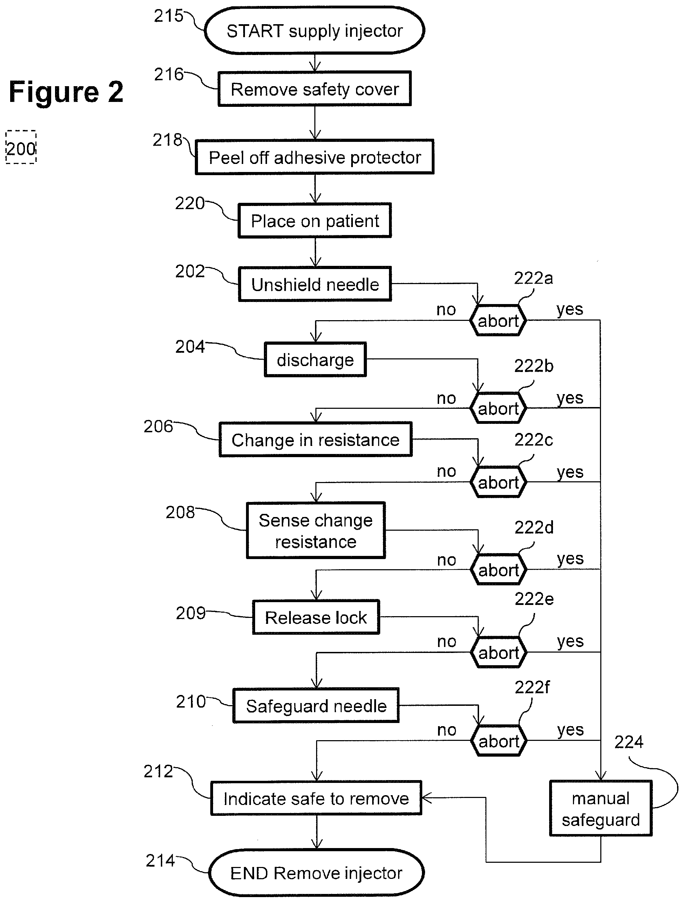

Referring now to the drawings, Fig. 2 illustrates a method of injecting a drug according to an embodiment **200** of the present invention. In the exemplary method, a needle is optionally retracted to a safeguarded position in response to a resistance to drug discharge. For example, the resistance may increase due to a malfunction such as an obstruction of a flow path and/or the injector completing injection of a full dose.

In exemplary embodiment **200** a user (for example a patient and/or a medical aid in home care) may be supplied **215** with an autoinjector ready to administer a medicine. The user may optionally remove **216** a safety cover from the injector. Removing **216** the cover may also automatically peel **218** an adhesive protector from an adhesive and/or remove a sterile cover from a needle. For example, the adhesive may be supplied to stabilize the injector on the skin of a patient during injection. The injector may optionally be placed **220** on a patient (who may be the user). In some embodiments a user may hold the injector to the skin of a patient, the adhesive may stabilize the injector for example from shifts and/or movements of the patient and/or the user.

In some embodiments, before injection, a needle may optionally be unshielded **202.** For example, while placing **220** the injector on the patient, the user may put pressure on an activation mechanism. The activation mechanism may react to pressure for example by unshielding **202** the needle, for example by extending the needle outward and/or inserting the needle into the patient. The needle may optionally be locked in the extended position. Optionally, the needle may be biased to a protected position (for example to retract into a housing of the injector). Alternatively or additionally, the needle may be biased to the unshielded position. Alternatively or additionally, the autoinjector may be supplied with the needle in an extended mode and/or protected by a cover.

At any point during the injection process, a manual safeguard **224** mechanism may be used to place the injector in a safeguarded mode. For example, if the user decides to abort **222a-f** at any point in the process (for example if he detects some sort of malfunction and/or feels a negative reaction to the medicine) the user may manually safeguard **224** the needle. For example, a needle shield may be released to manually safeguard **224** the needle and/or the needle may be retracted. Optionally there may be an indicator to indicate **212** whether the needle was automatically safeguarded **210** and/or whether needle was manually safeguarded **224.** Alternatively or additionally there may be an indicator whether a full dose was administered and/or how much medicine was administered.

Once the needle is inserted into the patient, the injector may optionally begin discharging **204** medicine. For example the medicine may be injected through the needle into the patient. Optionally, discharge may continue until a full dose of the medicine is administered.

In some embodiments, after administration of a full dose of the medicine, there may be a change **206** in resistance to further discharging. For example in a syringe based injector, a plunger may reach the end of the syringe and cease to move increasing resistance. Alternatively or additionally, after discharging the entire dose a transmission may be disconnected (for example a threaded element may pass the end of its threading) reducing resistance. Alternatively or additionally, the change **206** in resistance may result from another cause for example increased resistance due to a full or partial occlusion of a fluid pathway and/or jamming of a mechanical component (for example cross threading of a screw). The change of resistance may optionally be sensed **208** triggering safeguarding **210** of the needle.

In some embodiments, the needle may be locked in an unshielded **202** state by a force sensitive lock. When the lock senses **208** the change **206** in resistance, it may release **209** the needle which may be safeguarded **210** by moving the tip to a shielded position (for example by retracting the needle tip to a shielded location). Alternatively or additionally, safeguarding **210** may include covering the needle tip with a needle shield.

In some embodiments, a flag may be supplied (for example a LED and/or a changing color indicator) to indicate **212** to the user that the needle has been safeguarded **210** and/or that the injector can safely be removed **214** from the patient.

### 3 States of an autoinjector

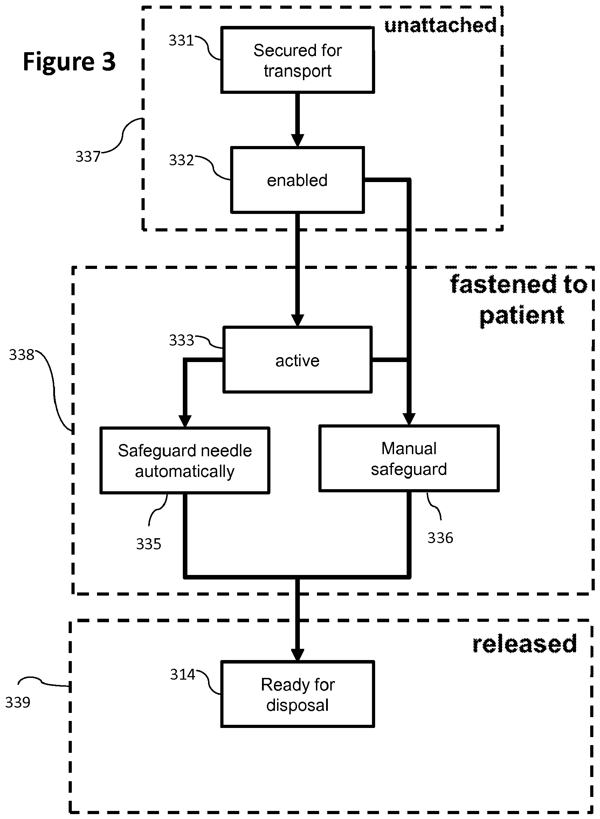

Fig. 3 is a state diagram of an autoinjector according to an embodiment of the present invention. In general, may be supplied in an unattached **337** state. An unattached **337** autoinjector may have a secured **331** state. For example in the secured **331** state the injector may be safe to handle and/or transport. Optionally the injector may have an enabled **332** state. For example, in the enabled **332** state, the injector may be unstable and/or easily activated. For example, an injector may be switched from the secured **331** state to the enabled **332** state by removing a needle protector and/or an adhesive cover.

Once activated the injector may optionally be fastened to a patient. In the fastened **338** state the injected may optionally be activated. For example, while the injector is in the active **333** state, a needle may project from the injector. In some embodiments the injector may be hazardous to handle in the enabled **332** and/or active **333** state.

In some embodiments, after use (optionally whether or not administration of the full dose was successful) the user may want to remove and/or dispose of the autoinjector. In some embodiments, it may be difficult and/or dangerous to remove an injector in the enabled and/or active state. For example, when an injector is fastened to a patient by an adhesive, it may be difficult to remove the needle by pulling the injector away from the skin. Optionally, first a needle may be retracted from the skin into the injector. Subsequently the adhesive may be removal by peeling from the skin. In some embodiments, the injector may automatically be safeguarded **335** for example by retraction of a needle upon completion of injection. Alternatively or additionally, the user may have the option to manually secure the injector into a safeguarded **336** state. For example, the optionally of manually needle retraction may avoid the situation where a patient may not be able to properly remove the injector due to a malfunction that leaves the injector fastened to the skin with the needle inserted into the patient. During and/or after safeguarding **335, 336** the injector may be removed from the patient.

Optionally, the injector may have a final released state **339,** for example wherein the needle is retracted back into the injector and/or the needle tip is shielded and/or the injector has been unfastened from the patient. Optionally one or more indicators may be supplied to indicate the state of the injector and/or the quantity of medicine discharged. Once released, the injector may be in final **314** state (protected from hazards and/or ready for disposal, for example in a municipal waste).

### 4 Needle safeguarding triggered by a linear force

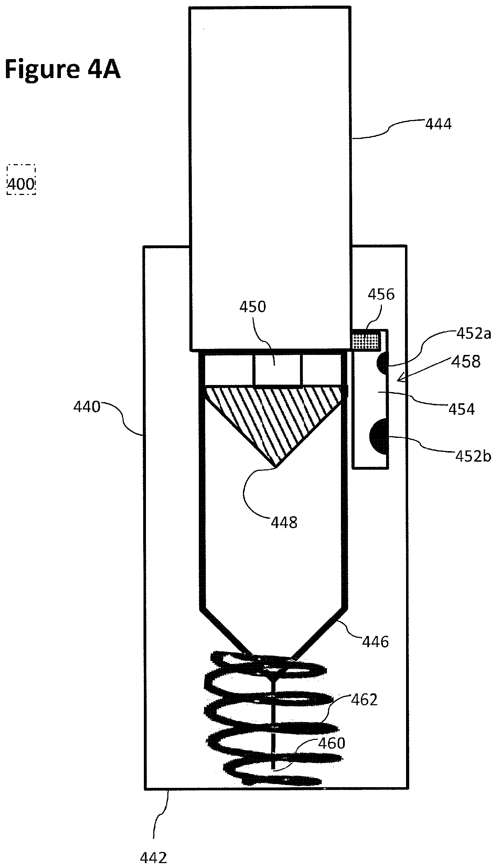

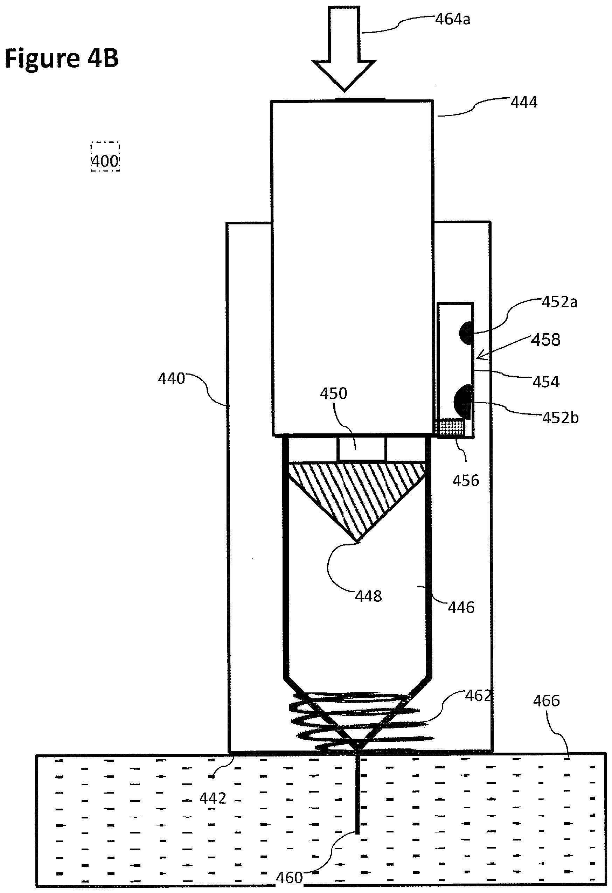

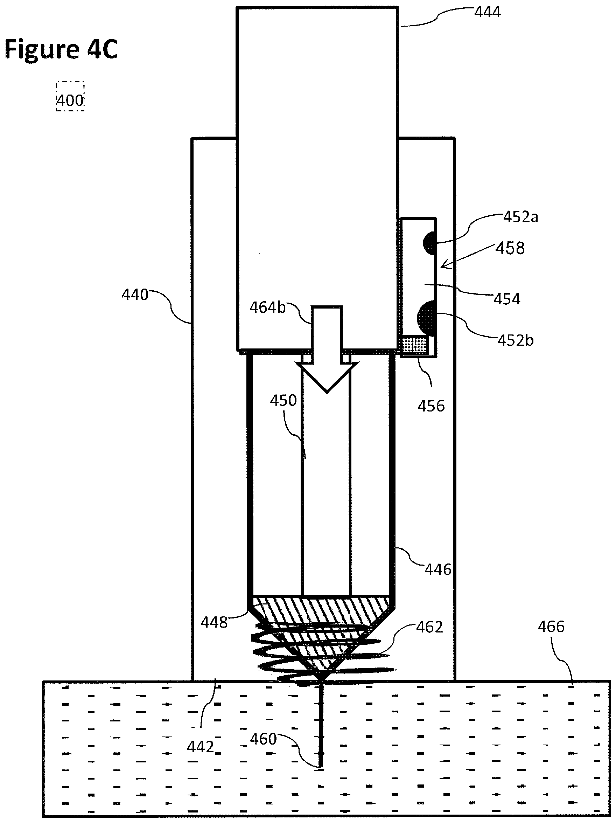

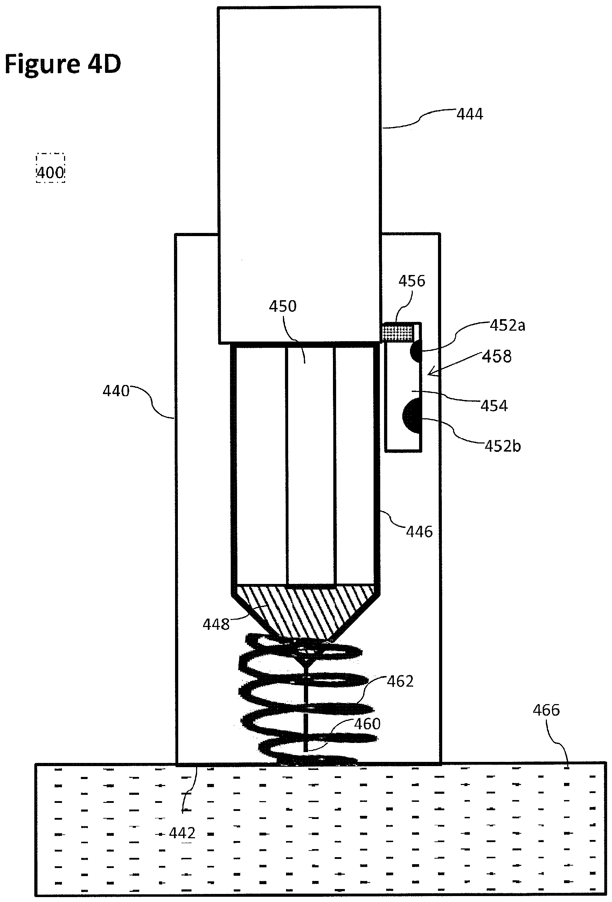

Figs. 4A-D are schematic illustrations of a needle safeguarding mechanism that is triggered by a linear resistance to discharge of a drug according to an exemplary embodiment **400** of the present invention. In exemplary embodiment **400,** a needle is biased to a retracted state for example by a spring **462.** During injection (for example as illustrated in Fig. 4B) the needle is optionally held in an unshielded state by a driver **444.** Driver **444** itself may be locked in place for example by an interference element **452b.** Additionally or alternatively driver **444** may push a plunger **448** to discharge a medicine. When a full dose has been administered (for example as illustrated in Fig. 4C), plunger **448** may optionally reach the end of its path and be stopped. For example plunger **448** may be stopped by the front end of a syringe **446.** Optionally syringe **446** may rest against a housing **440.** In some embodiments interference element **452b** may serve as a sensor. For example, the force of the driver pushing a plunger rod **450,** plunger **448** and/or syringe **446** against housing **440** may optionally overcome interference element **452b,** unlocking driver **444** and/or syringe **446** and/or the needle (for example as illustrated by force **464b** of Fig. 4C). Once unlocked, driver **444** may revert to a retracted state and/or the needle may optionally retract back into the housing (for example as illustrated in Fig. 4D).

In some embodiments, interference elements **452a,b** and spring **462** may be elastic and/or deformable, allowing them to be deformed by a sufficient force (for example as illustrated by force **464b** of Fig. 4C) to unshield the needle.

Figure 4A illustrates exemplary embodiment **400** in a deployed state prior to activation. For example, in the deployed state a needle tip **460** is shielded by an activation zone **442** of housing **440.** Needle tip **460** is safeguarded by a retraction assembly including for example a spring **462** biasing the needle into housing **440** and a lock **458** and a driver **444** which hold needle tip **460** and its supporting syringe **446** inside housing **440.** In exemplary embodiment **400,** lock **458** optionally includes a driver support **456** and a slot **454** and interference elements **452a** and **452b.** In Fig. 4A, driver **444,** syringe **446** and needle tip **460** are held in the retracted position by interference element **452a** and/or spring **462.**

Fig. 4B illustrates exemplary embodiment **400** in an activated state, for example right before and/or during discharge of a medication. For example, embodiment **400** is activated by placing activation zone **442** against the skin **466** of a patient and/or pushing driver **444** with a sufficient force **464a.** Pushing driver **444** optionally pushes needle tip **460,** syringe **446,** and/or driver support **456** from the retracted position (illustrated in Fig. 4A) to the extended position (illustrated in Fig. 4B). As support **456** moves from the retracted to the extended position, it slides along slot **454.** Sliding along slot **454,** support **456** overcomes biasing spring **462** and/or interference elements **452a,b.** Optionally support **456,** syringe **446** and/or driver **444** are locked into the unshielded position by interference element **452b.** Once activated, driver **444** may apply a force **464b** (see Fig. 4C) on plunger rod **450** and/or plunger **448** to discharge medicine.

Figure 4C illustrates exemplary embodiment **400** at the end of discharge of a medicament. Plunger **448** has optionally reached the end of its path and is encounters resistance from further movement. For example, further movement may be resisted by the front end of syringe **446** which is resting against housing **440.** Optionally the resistance force **464b** may be sensed by driver **444 as** a strong counter force pushing driver **444** and driver support **456** distally away from plunger **448.** The counterforce may optionally become large enough to overcome interference element **452b.** Overcoming interference element **452b** may optionally trigger safeguarding needle tip **460.** For example, biasing spring **462** and/or momentum may optionally overcome interference element **452a** returning support **456,** driver **444,** syringe **446** and/or needle tip **460** to the retracted safeguarded position (as illustrated for example in Fig. 4D).

### 5 Needle safeguarding by a telescoping element

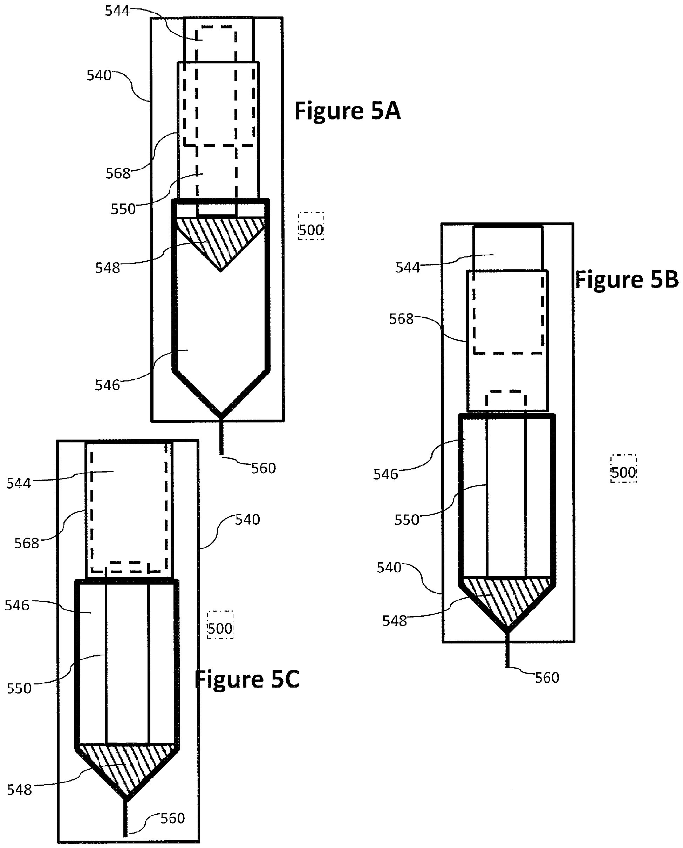

Figs. 5A-C are schematic side view illustrations of an autoinjector with a needle safeguarding mechanism including concentric cylindrical telescoping elements according to an exemplary embodiment **500** of the present invention. The safeguard mechanism of exemplary embodiment **500** includes a cylindrical driver **544** mounted concentrically in a cylindrical sleeve **568.** When driver **544** telescopes out from sleeve **568** a needle tip **560** is unshielded. When driver **544** retracts into sleeve **568,** needle tip **560** is safeguarded. As used herein the word cylindrical may include a cylinder with a circular and/or a non-circular cross section.

Fig. 5A illustrates the injector of exemplary embodiment **500** in an activated state before discharge of a medicine payload. Optionally, in the activated state, driver **544** is telescoped out of sleeve **568.** Driver 544 pushes, for example, against a housing **540.** Sleeve **568** optionally pushes a syringe **546** and/or needle point **560** extending needle point **560** out of housing **540** and/or unshielding needle point **560.**

Fig. 5B illustrates exemplary embodiment **500** at the end of medicine discharge. Driver **544** has optionally pushed a plunger rod **550** and/or plunger **548** into syringe **546** discharging the payload of the injector. In the example, further pushing by driver **544** will, for example, create a high stress between driver **544** and plunger rod **550.** The stress may optionally cause driver **544** to retract into sleeve **568.**

Fig. 5C illustrates that in exemplary embodiment **500,** when driver **544** retracted into sleeve **568** needle point **560** is optionally retracted back into a protected position. In the protected position, needle tip **560** may optionally be shielded by housing **540** safeguarding needle tip **560.**

### 6 Needle safeguarding triggered by a torque

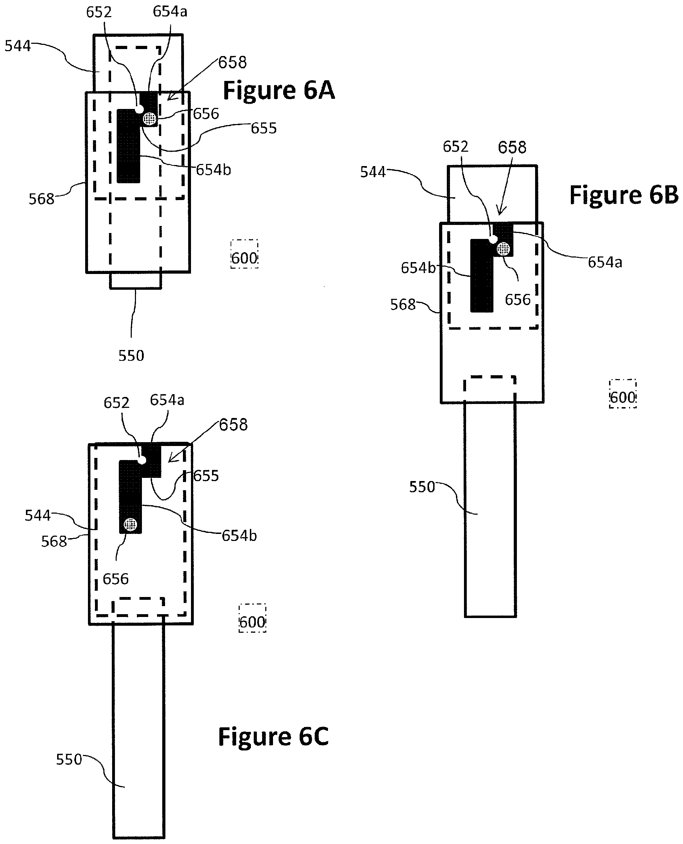

Figs. 6A-C are schematic side view illustrations of a lock **658** of a needle safeguarding mechanism triggered by a torque according to an embodiment **600** of the present invention. For example, in embodiment **600** lock **658** holds a needle (not shown) in an unshielded and/or extended state. Optionally a torque overcoming an interference element releases lock **658.** For example in embodiment **600,** when lock **658** is released, a driver **544** slides into a sleeve **568** retracting the needle to a safeguarded location.

The needle safeguarding mechanism of embodiment **600** may optionally include concentric cylindrical telescoping elements similar for example to embodiment **500.** Embodiment **600** is illustrated in Figs. 6A-C in states respectively similar to the states illustrated for embodiment **500** in Figs. 5A-C. Embodiment **600** may for example be used to hold a needle in an unshielded and/or safeguarded state in a manner similar to embodiment **500.**

Fig. 6A illustrates the safeguarding mechanism of exemplary embodiment **600** in an exemplary activated state before discharge of a medicine payload. Optionally, in the activated state, driver **544** is telescoped out of sleeve **568.** Driver **544** may optionally be connected to a support **656.** Support **656** may for example move along a doglegged slot having two tracks, for example track **654a** and track **654b.** In the extended state of Fig. 6A support **656** is blocked by a shoulder **655** of track **654a** optionally preventing driver **544** from sliding into sleeve **568.** Optionally, support **656** is blocked from moving to track **654b** by an interference element **652.** Optionally interference element **652** may be made of an elastic material that may be overcome by a sufficient stress.

Fig. 6B illustrates exemplary embodiment **600** at the end of medicine discharge. Driver **544** has optionally pushed a plunger rod **550** to the end of a syringe. Pushing of rod **550** may be for example by a telescoping screw. In the example of embodiment **600,** further pushing by driver **544** may, for example, create a high torque stress between driver **544** and plunger rod **550** and/or between driver **544** and sleeve **568.** The torque may for example overcome interference element **652** and slide support **656** onto track **654b.**

Fig. 6C illustrates that in exemplary embodiment **600,** after support **656** has been forced by torque onto track **654b.** Support **656** may slide along track **654b** and/or driver **544** may slide into sleeve **568.** Sliding driver **544** into sleeve **568** may optionally revert driver **544** and/or sleeve **568** and/or the needle to a retracted state and/or retract needle point **560** back into a protected position for example as illustrated in Fig. 5C.

In some embodiments there may be biasing element, for example a spring, biasing the needle to a safeguarded state. Optionally, embodiments **500** and/or **600** may contain a biasing element, for example similar to spring **462.**

### 7 An extended area rotational interference element

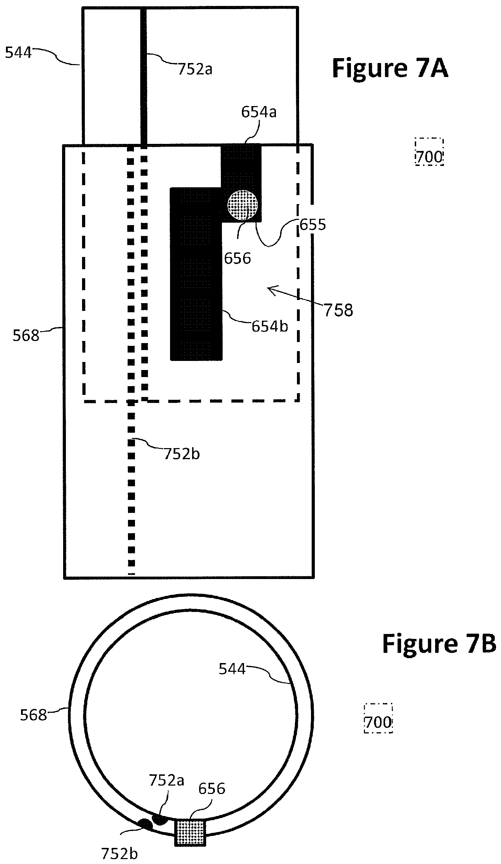

Figs. 7A,B illustrate a schematic side view and a top cross-sectional view respectively of a needle safeguarding mechanism triggered by a torque according to an embodiment **700** of the present invention. Embodiment **700** includes elongated interference elements **752a,b** resisting rotation of driver **544** with respect to sleeve **568.** The elongated interference elements **752a,b** spread the resistance to rotation over a large area. The large interference elements **752a,b** may in some embodiments produce a consistent resistance even if there are small flaws during formation of part elements **752a,b.**

Similar to embodiment **600,** embodiment **700** includes two concentric cylindrical telescoping elements, a driver **544** in a sleeve **568.** A lock **758** includes a driver support **656** that moves between two tracks **654a,b.** Lock **758** may optionally move from a locked state (holding the system in an extended mode) to a released state in response to a torque twisting driver **544** with respect to sleeve **568.** For example twisting may trigger retraction of a needle. For example twisting may be triggered by a plunger reaching the end of a syringe after administering a full dose of a drug. Alternatively or additionally retraction may be triggered by an obstruction in a fluid pathway providing resistance to movement. The resistance to movement of a screw thread plunger may optionally produce the torque to twist and/or unlock the retractor.

One difference between embodiment **600** and embodiment **700,** is the location and geometry of the resistance interference elements. In **700** the resistance interference elements **752a,b** include two ribs, one rib (element **752a**) located on an outer surface of driver **544** and another rib (element **752b**) located on an inner surface of sleeve **568.** Resistance interference elements **752a,b** resist rotation of a driver **544** within sleeve **568** and/or movement of support **656** from track **654a** to **654b.** When support **656** is on track **654a,** driver **544** and/or the needle may remain in an extended position. When support **656** is on track **654b,** driver **544** and/or the needle may be retracted. Alternatively or additionally there may be more than two ribs serving as resistance elements. For example, multiple pairs of ribs may engage simultaneously to create a compound resistance at a single point of rotation. Alternatively or additionally, there may be more than two sections of tracks and or there may be multiple steps of resistance and/or extension and/or retraction.

### 8 Multi-conditional needle safeguarding

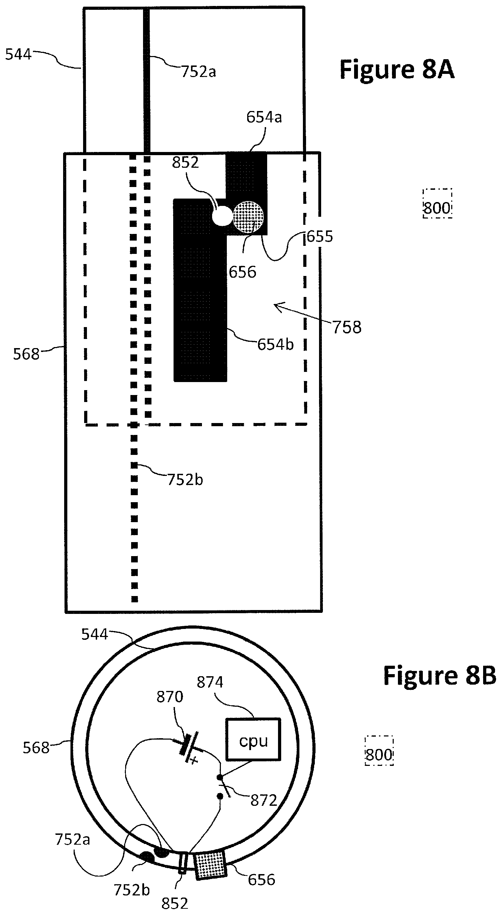

Figs. 8A,B illustrate a schematic side view and a top cross-sectional view respectively of a needle safeguarding mechanism triggered by a torque and a second condition according to an embodiment **800** of the present invention. Embodiment **800** includes a logical sensor. For example a logic controlled interference element **852** resists rotation of driver **544** with respect to sleeve **568.** Interference element **852** may optionally be responsive to a controller **874.** In some embodiments even when there is a torque capable of overcoming interference elements **752a,b** the needle will not retract until controller **874** opens interference element **852.** For example, controller **874** may open resistance element **852** when a predetermined portion (for example substantially all of) a payload of the injector has be administered. Optionally, the needle will be retracted when there is a resistance to discharge of the medicine and the entire payload has been administered. Optionally the needle may not retract in response to an obstruction to the fluid pathway when it occurs before the entire payload is administered.

Embodiment **800** may be similar in many aspects to embodiment **700.** Embodiment **800** differs from embodiment **700,** at least in that embodiment **800** includes an additional interference element **852.** Optionally, clement **852** blocks retraction of a needle until a further condition is fulfilled (for example in embodiment **700** the needle may retract on any application of a sufficient torque to driver **544** whereas in embodiment **800** the needle may optionally be prevented from retracted until a sufficient torque is applied and the payload of the injector has been sufficiently administered).

In some embodiments logic controlled resistance element **852** may block movement of support **656** to track **654b.** When the sufficient portion of the payload of the injector has been administered, controller **874** may close a switch **872.** Closing switch **872** may optionally allow a current from a battery **870** to flow to element **852,** melting element **852** and/or allowing support **656** to move to path **654b** and/or allowing driver **544** to slide into sleeve **568.**

### 9 Detailed illustration of states of an injector

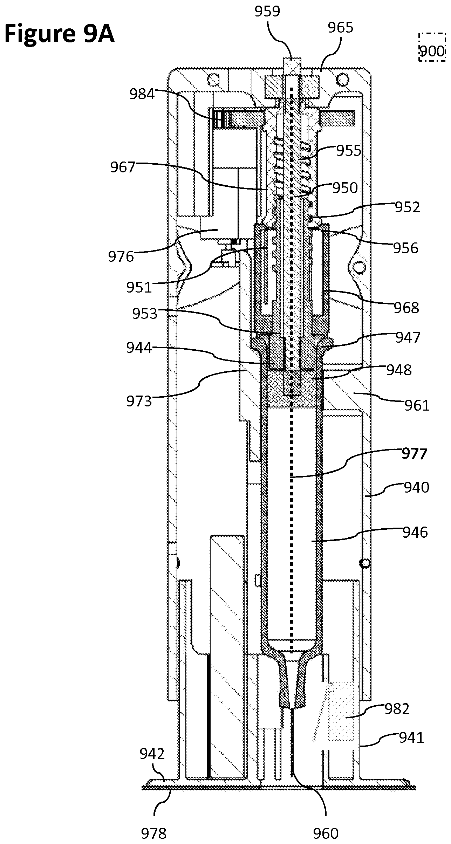

Figs. 9A-D include detailed cross sectional side views illustrating four states of an autoinjector according to an embodiment of the present invention. In some embodiments, an injector **900** is an automated self injection device. For example the self injecting device may in some ways be similar to a pen injector. Optionally injector **900** may be loaded with a standard type syringe **946** and/or hypodermic needle **960.** For example, needle **960** may be rigidly connected and/or project from a distal end of syringe **946.** Needle **960** may be coaxial with syringe **946.** Alternatively or additionally the axis of needle **960** may be parallel to the primary longitudinal axis **977** of syringe **946** but offset therefrom. Syringe may be loaded into injector 900 with needle **960** in a sterile state and/or covered by a sterile cover.

In some embodiments, an injector may include for example an adhesive **978** base **942.** For example, adhesive **978** base **942** may assist a user to hold injector **900** steady on the skin of a patient for an extended period. For example, injector **900** may be used to give injections of volume ranging between 0.5 and 3.0 ml over a time period ranging between 30 sec to 180 sec.

Injector **900** includes for example an annular snap resistance element **952** paired to an annular driver support **956.** When a linear stress increases past a threshold, the annular snap gives way and a needle **960** may optionally be retracted to a protected location.

Fig. 9A is a schematic cross sectional side view illustrating injector **900** in an enabled state (ready for activation). For example, in the enabled state an optional safety cover and/or a sterile cover may and/or an adhesive protector may have been removed from the injector. In the enabled state needle **960** is in a protected location, created by a shield **941** which extends the distal end of a housing **940** of the injector. Needle **960** and/or shield **941** may optionally be retained in position, for example by a snap and/or held in position by a biasing device, for example a spring.

In some embodiments, needle **960** may optionally be supported by a syringe **946;** which is in turn supported for example by a cylindrical outer sleeve **968.** Outer sleeve **968** may optionally be supported by an annular support **956** resting on an annular snap resistance element **952.** For example annular snap resistance element 952 may extend radially outward from a cylindrical inner sleeve **967.** Optionally, inner sleeve **967** and/or outer sleeve **968** and/or a driver **944** may be operationally linked to a transmission **984** such that rotating transmission **984** rotates one or more of inner sleeve **967** and/or outer sleeve **968** and/or a driver **944.**

In some embodiments, a motor switch **982** may be located in shield **941.** In the enabled state (before activation), switch **982** is optionally switched off.

In injector **900,** syringe **946** is held to outer housing **940** by a socket **961.** Socket **961** allows syringe **946** to slide axially with respect to housing **940** but not to move laterally. In injector **900,** transmission **984** is held rotatably fixed to housing **940** by bearing **959** in a hub **965.**

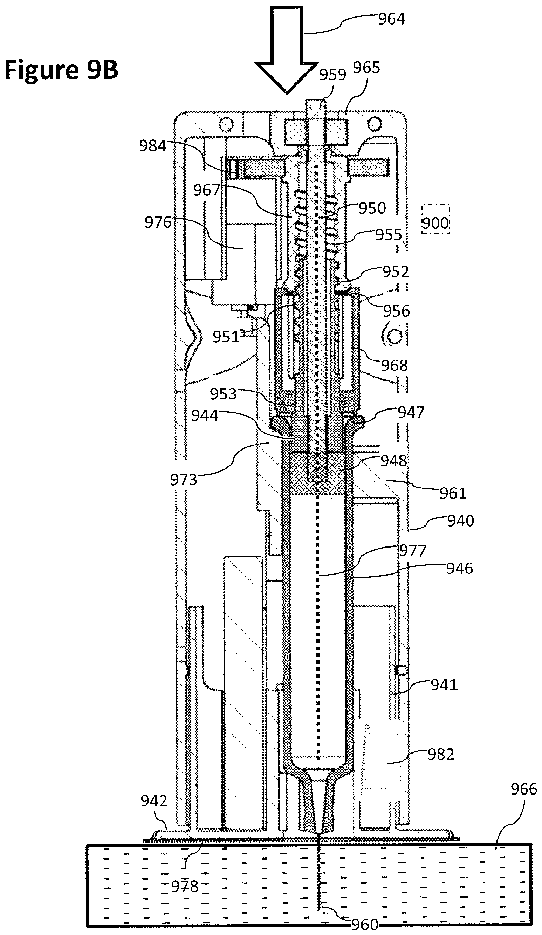

Fig. 9B is a schematic cross sectional side view illustrating injector **900** immediately after activation. For example, to activate the injector, a user may place the distal end of the injector (including for example an adhesive **978** and/or an activation zone on base **942)** against the skin **966** of a patient and/or push **964** on the proximal end of the injector until shield **941** collapses into housing **940** in a direction parallel to the longitudinal axis of needle **960.** Collapse of shield **941** may optionally unshield needle **960** tip which may for example be pushed into the skin **966** of the patient. For example, in operation, needle **960** may protrude from injector **900** into a patient. Optionally, in operation, needle **960** may be in fluid communication with syringe **946** and/or the patient. For example needle **960** may supply a fluid pathway for discharging medicine directly from syringe **946** through needle **960** into the patient.

In some embodiments, collapse of shield **941** may activate switch **982.** For example in injector **900** switch **982** is depressed by being pushed against syringe **946.** Depressing switch **982** may activate a motor **976** to start discharging a drug. For example, in injector **900** motor **976** turns a transmission **984.** Transmission **984** may include for example a gear. Transmission **984** may optionally rotate inner sleeve **967** and/or driver **944.** In exemplary injector **900,** driver **944** includes teeth and/or threads which engage a screw thread **953** on a plunger rod **950.** Rotating driver **944** may optionally drive plunger rod **950** and/or plunger **948** in the distal direction, discharging the medicine. Optionally, plunger **948** continues to move distally until it is stopped by for example a blockage in the fluid path (preventing further discharge) and/or until plunger **948** reaches the distal end of syringe **946.** Optionally, when needle **960** is in the extended position, a flange **947** of syringe **946** seats against a bracket **973,** which holds syringe **946** and/or prevents further longitudinal movement.

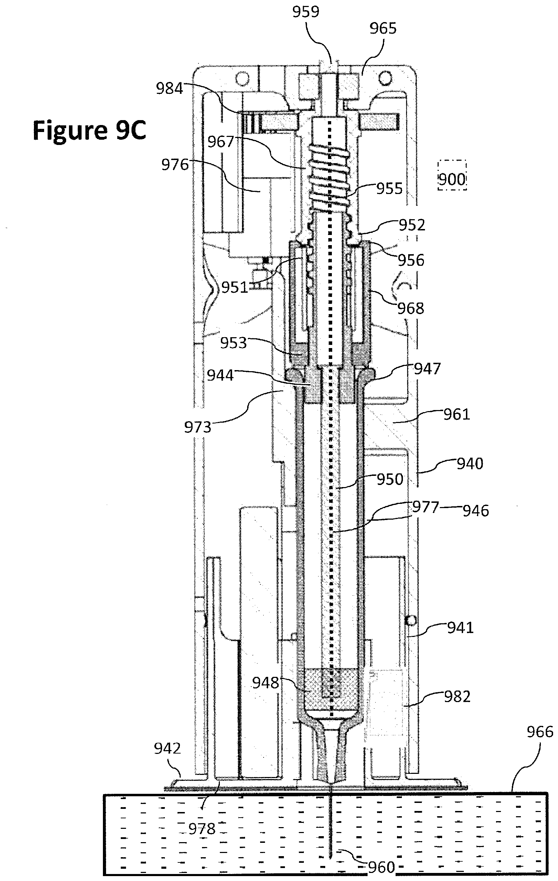

Fig. 9C is a schematic cross sectional side view illustrating injector **900** at the end of discharge of the payload. For example plunger **948** has discharged all of the medicine out of syringe **946** and/or has reached the distal end of syringe **946.** Optionally, further rotation of driver **944** increases the stress pushing driver **944** proximally. Interference element **952** may serve as stress sensor. For example, motor **976** may supply enough torque to create a force which overcomes interference element **952.**

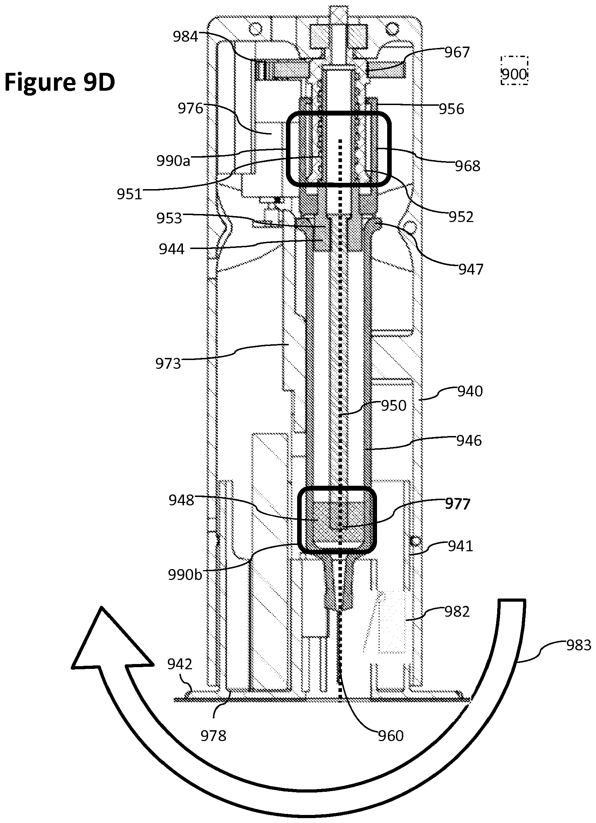

In some embodiments, once interference element **952** is overcome a course inner threading **955** in sleeve **967** rotates with respect to a course outer thread **951** of driver **944** drawing driver **944** and/or plunger **948** and/or syringe **946** and/or needle **960** proximally into a retracted state. For example, the course thread **951** has an opposite threading from the threading **953** between driver **944** and plunger rod **950.** The same direction of rotation that drives plunger **948** distally before overcoming interference element **952** also draws back plunger **948** and/or syringe **946** and/or needle **960** proximally after overcoming interference element **952.** Optionally needle **960** is retracted into a protected location inside housing **940** for example as illustrated in Fig. 9D. Alternatively or additionally, course thread **951** may have an the same direction of threading as threading **953** between driver **944** and plunger rod **950** and optionally rotation may be reversed to retract needle **960.**

Fig. 9D is a schematic cross sectional side view illustrating injector **900** in a safe state after finishing injection. Needle **960** point has optionally been retracted into a protected location within housing **940.** Syringe **946** has optionally been retracted. In exemplary injector **900,** when syringe **946** is retracted, it no longer depresses switch **982.** Switch **982** may be biased off and/or raising syringe **946** may shut off motor **976.**

In some embodiments one or more windows may be supplied. A user may be able to determine a status of the device by viewing for the windows. For example in Fig. 9D, injector 900 has been supplied with two windows **990a,b.** For example window **990a** is located such that during injection, the user views inner sleeve **967** through window **990a.** When outer sleeve **968** has been retracted, it may optionally slide over inner sleeve **967.** After outer sleeve **968** has been retracted, the user views outer sleeve **968** through window **990a.** Optionally window **990a** may serve as an indicator whether it is safe to remove the injector. For example, outer sleeve **968** may be colored green and/or driver **944** and/or inner sleeve **967** may be colored red. For example, as long as the user sees red in window **990a** needle **960** tip has not been retracted and/or it is unsafe to remove the injector from the patient's skin; and/or when the user views green through window **990a** needle **960** has been retracted and/or discharge has ceased and/or it is safe to remove the injector from the skin of the patient. Optionally, window **990b** may be used to indicate whether an entire payload of medicine has been administered. For example, syringe **946** may be made of a transparent material. For example, during injection, the user can see the medicine through window **990b;** after syringe **946** is retracted if the payload has been fully discharged then the user will view plunger **948** through window **990b.** Optionally, if the user sees plunger **948** through both window **990b** and outer sleeve **968** through window **990a** then the user can ascertain that it is safe to remove the injector and/or that the drug was fully discharged.

In injector **900,** for example, after retraction of the needle the device may be twisted such that one side of the adhesive is lifted and/or peeled (as illustrated by arrow **983** in Fig. 9D) from the skin while the far edge of the base of the injector remains in contact with the skin and serves as a fulcrum.

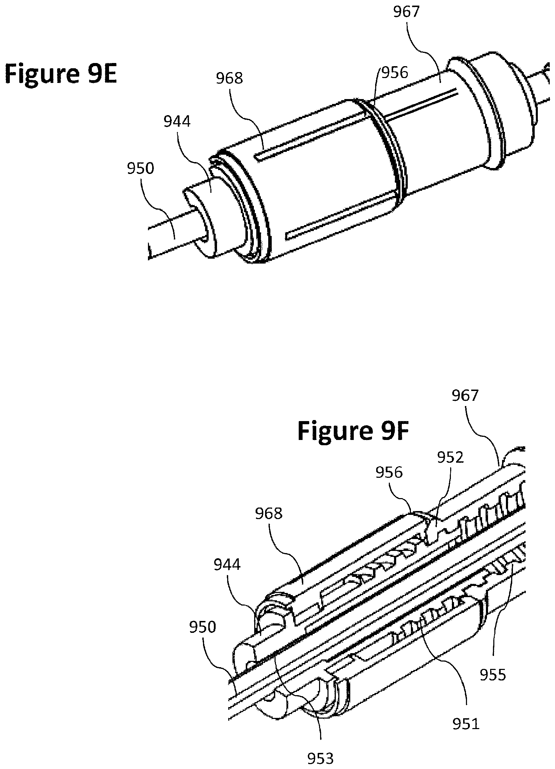

Figs. 9E and 6F illustrate an external view and cut away view respectively of a needle retractor according to exemplary injector **900** of the current invention. Injector **900** may optionally be designed such that, under sufficient linear stress, support **956** of external sleeve **968** deforms and/or opens to pass over resistance element **952.** Optionally, transmission **984** and/or inner sleeve **967** may be formed of one or more pieces of molded plastic. Optionally outer sleeve **968** and/or driver **944** may be formed of one or more pieces of molded plastic.

Fig. 9F, illustrates details of a rotary needle retractor according to an embodiment of the current invention. Fig. 9F illustrates driver **944** before needle retraction (for example in a secured state, an enabled state and/or an active state). In the exemplary embodiment, driver **944** is engaged by a set of fine screw threads **953** to rod **950.** In the exemplary embodiment, driver **944** is engaged by a set of course screw threads **951, 955** to inner sleeve **967.** Optionally, course screw threads **951, 955** are threaded in an opposite sense from fine screw threads **953.**

In the exemplary embodiment, prior to needle retraction, sleeve **967, 968** and driver **944** are prevented from sliding longitudinally with respect one another. While sleeves **967, 968** and driver **944** are prevented from relative longitudinal movement, threads **951** and **955** prevent inner sleeve **967** and driver **944** from rotating with respect to one another.

In some embodiments, motor **976** drives transmission **984** to rotate inner sleeve **967.** Optionally, before needle retraction, rotating inner sleeve **967** rotates driver **944.** The sense of screw threads **953** and the rotating direction of motor **976** are optionally chosen such that rotating driver **944** relative to rod **950** pushes rod **950** and/or plunger **948** distally, optionally discharging a drug.

When plunger **948** has reached the distal end of syringe **946,** rod **950** is prevent from further distal movement. Torque applied to driver **944** produces a strong proximal stress on driver **944** and/or outer sleeve **968.** The strong proximal stress overcomes and/or releases interference element **952.** Once interference element **952,** is released outer sleeve **968** and/or driver 944 can move longitudinally with respect to inner sleeve **967.** Further rotation of inner sleeve **967** rotates sleeve **967** with respect to driver **944.** The sense of screw threads **955** and **951** and the rotating direction of motor **976** are optionally chosen such rotating driver **944** relative to sleeve **967** draws driver **944** and/or rod **950** and/or plunger **948** and/or syringe **946** and/or needle **960** proximally, optionally retracting needle **960.** Optionally the pitch of screw threads **951, 953** and/or **955** can be tuned to achieve a desired rate of medicine discharge and/or needle retraction for a given rotation rate of the motor. In some embodiments, as rod **950** and/or plunger **948** are drawn proximally, friction between plunger **948** and syringe **946** draws syringe **946** and/or needle **960** proximally. Alternatively or additionally, outer sleeve **968** may be attached to syringe **946.** Drawing back on driver **944** may draw outer sleeve **968** and syringe **946** back with it. In some embodiments additional threaded elements may be added to produce a multi-part telescoping assembly for extending plunger **948** to discharge medicine and/or for retracting needle **960.** In some embodiments some or all of rod **950,** inner sleeve **967,** and/or outer sleeve **968** and/or transmission **984** may be formed of molded plastic and or other materials.

### 10 Stabilized pen injector

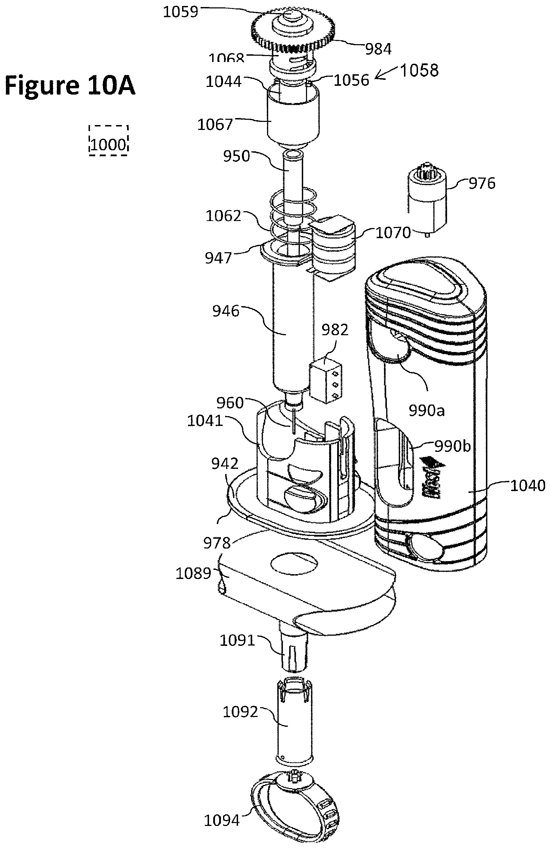

Figs. 10A-H illustrate a stabilized injector **1000** according to some embodiments of the present invention. Exemplary injector **1000** is an automated injection device in some ways similar to a pen injector. Optionally injector **1000** may be loaded with a standard type syringe **946** and/or hypodermic needle **960.** Optionally syringe **946** may be supplied loaded with medicine and/or covered with a sterile needle cover **1091.** Syringe **946** may be loaded into injector **1000** with in a sterile state with needle cover **1091** in place. Injector **1000** may include for example an adhesive **978** base **942.** In some embodiments, adhesive **978** base **942** may assist a user to hold injector **1000** steady on the skin of a patient for an extended period. For example, injector **1000** may be used to give injections of volume ranging between 0.5 and 3.0 ml over a time period ranging between 30 sec to 180 sec.

Fig. 10A illustrates an exploded view of injector **1000.** Some components of the exemplary embodiment of injector **1000** which are similar to corresponding parts of the exemplary embodiment of injector **900** are marked with the same number as the corresponding parts of the exemplary embodiment of injector **900.**

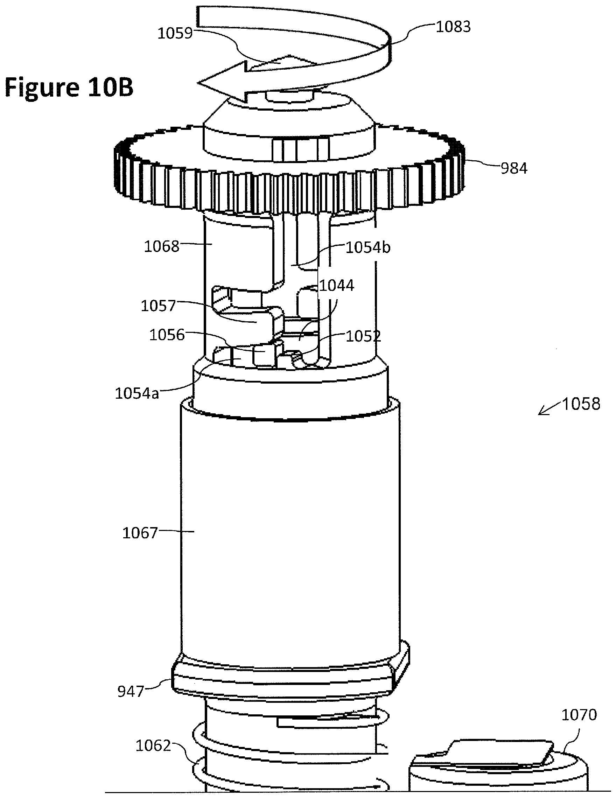

In the exemplary embodiment of injector **1000** a power supply (for example batteries **1070**) may optionally supply power to gear motor **976.** Figs. 10A,B illustrate flange **947** of syringe **946.** Optionally flange **947** has at least one non-rounded edge which may be held inside an autoinjector (for example autoinjectors **400, 900** and/or **1000**) preventing rotation of syringe **946.** Outer housing **1040** and/or shield **1041** of injector **1000** are similar to outer housing **940** and/or shield **941** of injector **900.**

Some embodiments of a stabilized autoinjector (for example as illustrated in injector **1000** but optionally included in injectors **900** and/or embodiments **400** and/or **200** and/or **100**) may include a safety cover and/or an adhesive protector and/or a handle.

Fig. 10B illustrates exemplary retraction mechanism **1058.** Retraction mechanism **1058** is optionally activated by a combination of torque and linear stress. For example retraction mechanism **1058** is optionally activated may when plunger **948** is blocked for example when it reaches the end of injection (for example as described in regards to Figs. 9A-D and/or due to an occlusion of needle **960**).

In some embodiments, during drug discharge a motor (for example motor **976**) rotates transmission **984** in the direction of arrow **1083.** Transmission **984** may optionally be rigidly connected to and/or integrally molded with inner sleeve **1068.** Rotating transmission **984** may also rotate inner sleeve **1068.** A pin **1056** protrudes from driver **1044** into a nearly lateral slot **1054a in** sleeve **1068.** While pin **1056** is in slot **1054a,** driver **1044** is prevented from moving longitudinally with respect to inner sleeve **1068.** In some embodiments syringe **946** is supported (from moving proximally) by driver **1044.**

In some embodiments, when there is a strong linear force on driver **1044** in the proximal direction and/or there is a strong torque on sleeve **1068** in the direction of arrow **1083,** arm **1057** is deflected upward and pin **1056** slides past an interference element **1052** into a longitudinal slot **1054b.** In slot **1054b** pin **1056** may slide longitudinally (in the proximal direction). A geometry of pin **1056** and/or interference element **1052** may be chosen to achieve a desired resistance to movement. For example, pin **1056** and/or interference element **1052** may have a squared side, a flat side, a rounded side etc.

In some embodiments, a spring (for example spring **1062**) biases syringe **946** in the proximal direction. For example spring **1062** may apply a proximal force to flange **947.** Optionally another biasing element may be used in place of spring **1062.** For example, a biasing element may include a stretched element (for example a rubber band and/or a twisted elements and/or a deflected plastic element).

Optionally when pin **1056** enters longitudinal slot **1054b,** spring **1062** pushes syringe **946** and/or outer sleeve **1067** and/or needle **960** and/or driver **1044** and/or pin **1056** proximally, retracting needle **960.** Optionally, needle **960** may be held in the retracted position by spring **1062.** Alternatively or additionally a locking mechanism may be included to lock needle **960** in the retracted position, for example, a one way catch and/or an interference element may lock against syringe **946** as it is retracted and/or against pin **1056** in slot **1054b.** Optionally, in injector **1000** driver **1044** includes two molded plastic telescoping pieces. One piece is optionally integrally molded with outer sleeve **1067.** Optionally, sleeve **1067** and/or driver **1044** may be made as a single piece and/or multiple parts. They may be formed of plastic and/or another material and/or they may be molded and/or formed by another process.

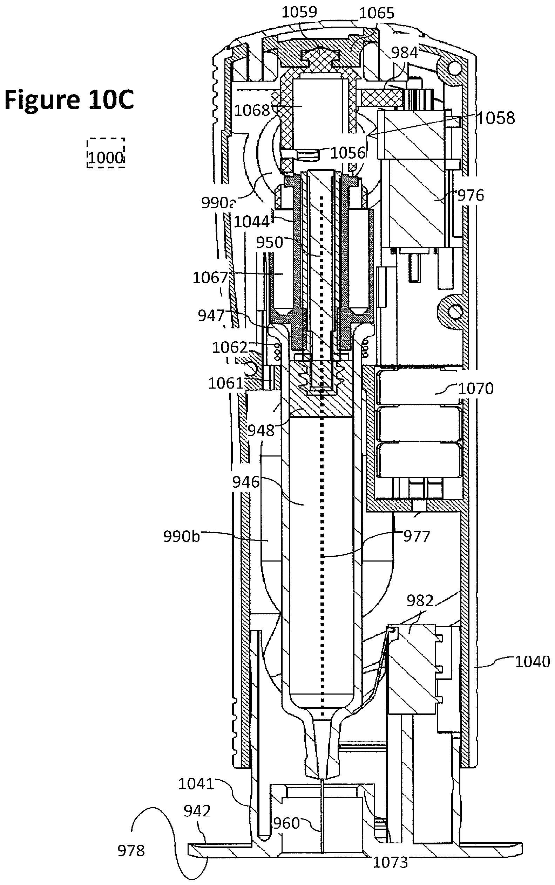

Fig. 10C illustrates exemplary embodiment **1000** assembled and/or in an enabled state before insertion of needle **960** into a patient. Figure 10C illustrates various optional details and/or supporting structures for syringe **946** and/or plunger.

In injector **1000,** syringe **946** is held to outer housing **1040** by a socket **1061.** Socket **1061** allows syringe **946** to slide axially with respect to housing **1040** but not to move laterally. In injector **1000.** transmission **984** is held rotatably fixed to housing **1040** by bearing **1059** in a hub **1065.**

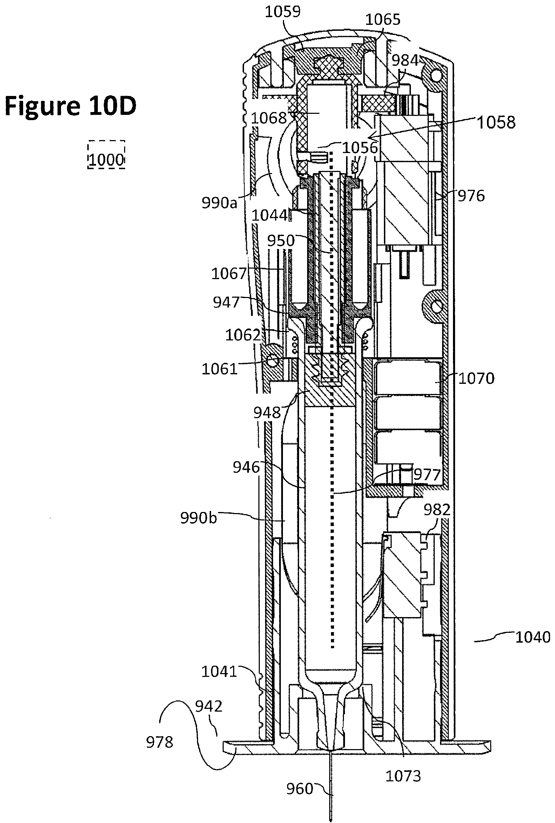

Fig. 10D illustrates exemplary injector **1000** in an active state. For example when the injector is in the enabled state, a user may place adhesive against the skin of a patient and push downward (distally) on housing **1040.** Housing **1040** and its contents (for example syringe **946,** transmission **984,** locking assembly of retraction mechanism **1058** etc.) along with needle **960** are all pushed distally along the axis of needle **960.** As needle **960** moves distally, the needle tip passes through a hole in shield **1041.** For example, in operation, needle **960** may protrude from injector **900** into a patient. Optionally, in operation, needle **960** may be in fluid communication with syringe **946** and/or the patient. For example needle **960** may supply a fluid pathway for discharging medicine directly from syringe **946** through needle **960** into the patient.

In some embodiments, when needle **960** is in the extended position, the front end of syringe **946** seats into a bracket **1073.** Bracket **1073** may optionally hold syringe **946** steady and/or prevents further longitudinal movement and/or prevent lateral movement with respect to housing **1040.**

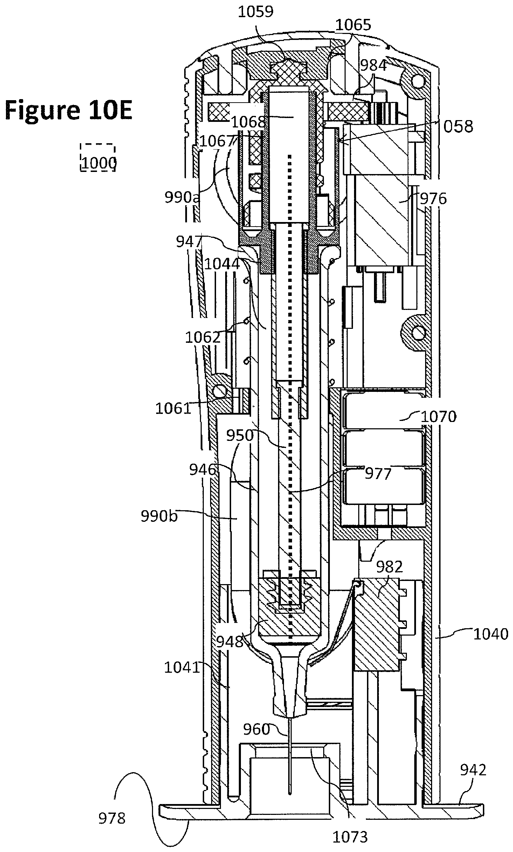

Fig. 10E illustrates injector **1000** after needle retraction. Optionally driver **944** includes a telescopic assembly, which is shown in an extended state in Fig. 10E. Optionally, after retraction of needle **960,** the entire device may be twisted to peel adhesive **978** from the skin.

Various aspects or features illustrated herein with respect to a particular embodiment may be combined with other embodiments. For example, needle **460** and **560** of embodiment **400** and **500** are shown mounted at an angle to base and/or activation zone **442** or **542.** Alternatively or additionally they may be perpendicular to the base. For example, needles **960** of embodiments **900** and **1000** are shown perpendicular to base **942.** Alternatively or additionally they may be at an angle to the base. Needle covers and/or protective covers illustrated in one embodiment may be used with another embodiment. Retraction mechanisms illustrated in one embodiment may be used with another embodiment. A clip, an interference element, a catch and/or another locking mechanism may hold an injector in one or another state. For example an interference element may hold a needle in a retracted position and/or in the extended position.

### 11 Manual needle safeguard

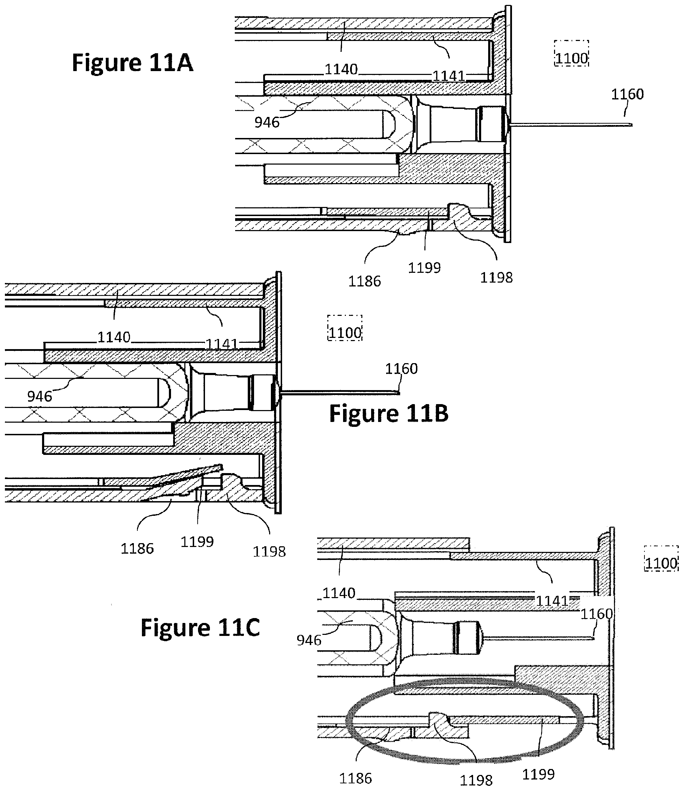

Figs. 11A-C illustrate a manual trigger for a needle protector of an autoinjector according to an embodiment **1100** of the present invention. In some embodiments a user may want to abort administration of a drug by an autoinjector and/or safeguard a needle of the autoinjector (for example when the automatic safeguard mechanism fails). Embodiment **1100** supplies a manual system for triggering a needle shield at a user's discretion.

Fig. 11A illustrates an autoinjector in an activated state. In the active state, a needle tip **1160** may be unshielded. For example, in Fig. 11A needle tip **1160** is shown extending out the proximal end of the injector. Optionally, a needle shield **1141** may be pushed distally into a housing **1140** of the injector unshielding needle tip **1160.** For example, in embodiment **1100** shield **1141** is held back in housing **1140** by a catch **1199** abutting against the distal side of a latch **1198.**

Fig. 11B illustrates initiation of manual release of a needle shield according to an embodiment **1100.** For example, a user presses inward on a needle guard release 1186. Optionally, pushing needle guard release **1186** disengages catch **1199** from latch **1198.** For example, needle shield **1141** may be biased to deploy outward (proximally) when catch **1199** is disengaged. The pressing needle guard release **1186** extends the needle guard and/or housing around the needle, retracting the needle point into a shielded location.

Fig. 11C illustrates an exemplary result of manual extension of the housing and/or retraction of the needle in exemplary embodiment **1100** of the present invention. Needle shield **1141** is shown in an exemplary deployed state. For example catch **1199** may abut on the proximal side of latch **1186** locking shield **1141** in the deployed state. Optionally, needle tip **1160** is safeguarded in a protected space surrounded by deployed needle shield **1141.**

### 12 Rotation resistance

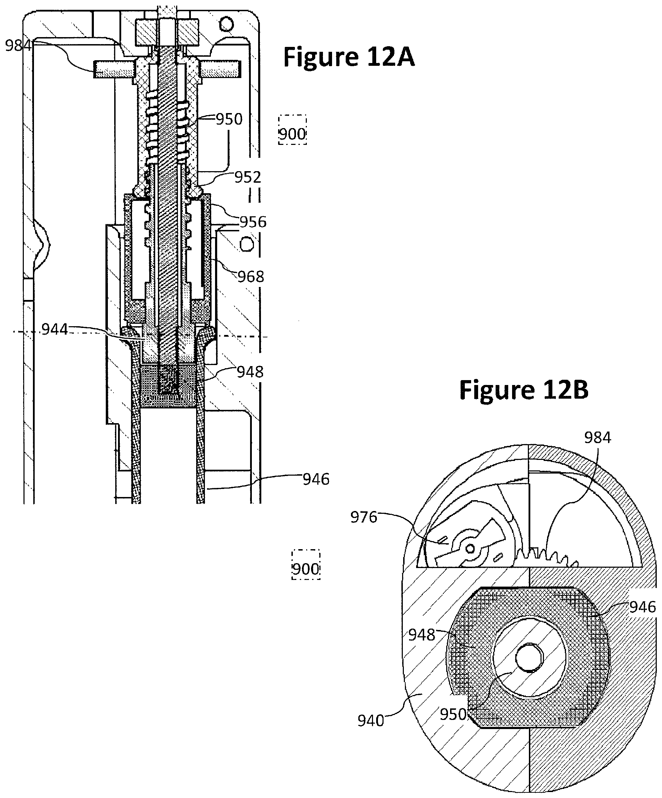

Figs. 12A,B illustrate an optional non-circular cross section for syringe **946** according to an embodiment of the present invention. Optionally syringe **946** may be prevented from rotating for example by giving it a non-circular cross section.

Fig. 12A is a side view of embodiment **900** showing a cutting plane for the cross sectional view of Fig. 12B.

Fig 12B is a cross section view of embodiment **900** illustrating and optional non-circular cross section of syringe **946.**

### 13 Microswitch activator for an autoinjector

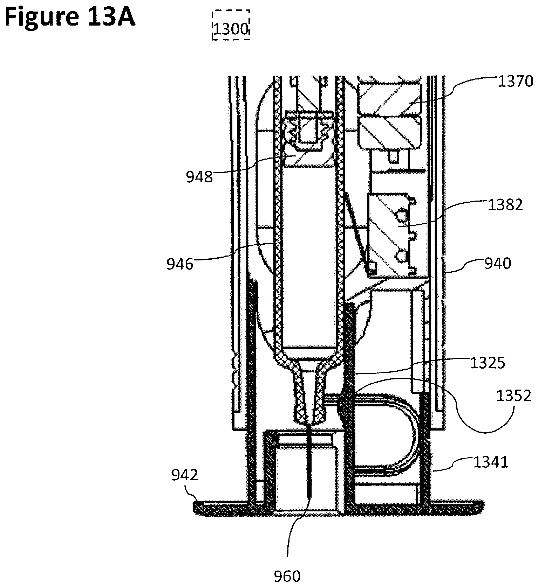

Figures 13A-D is are cutaway illustrations of an exemplary embodiment of an autoinjector **1300** with a motor switch located in the main body of the injector in accordance with some embodiments of the current invention. Optionally the motor switch remains stationary with respect to the housing of the autoinjector, for example housing **940,** and/or with respect to and/or a motor and/or a power source, for example batteries **1370.** When an activator of the injector is triggered (for example by collapsing a shield **1341**), movement of the activator may activate the switch. When the needle **960** is retracted and/or shielded (for example after injection), the switch is optionally returned to the inactivated state. Keeping a switch stationary with respect to a motor and/or power source may simplify assembly of the auto injector.

FIG 13A illustrates injector **1300,** prior to activation in accordance with an embodiment of the current invention. Optionally many structures are the same as other embodiments listed herein above or below, for example, injector **1300** may include the advancement and/or retraction mechanism of injector **900.** Optionally, prior to activation, switch **1382** is in a deactivated position.

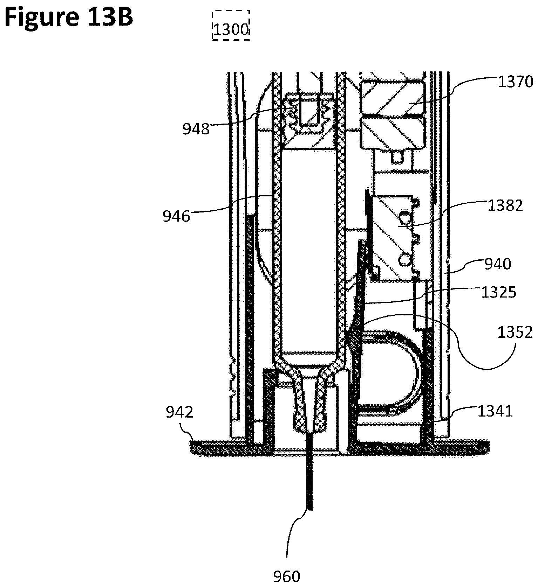

FIG 13B illustrates injector **1300,** immediately after activation in accordance with an embodiment of the current invention. In some embodiments, an injector may be activated by collapsing a needle shield **1341.** Collapsing shield **1341** optionally exposes needle **960** and/or pushes an element **1352** against a side of a syringe and/or pushes an extension **1325** of shield **1341** into switch **1382** and/or activates switch **1382** and/or activates a motor (for example similar to motor **976** of injector **900**).

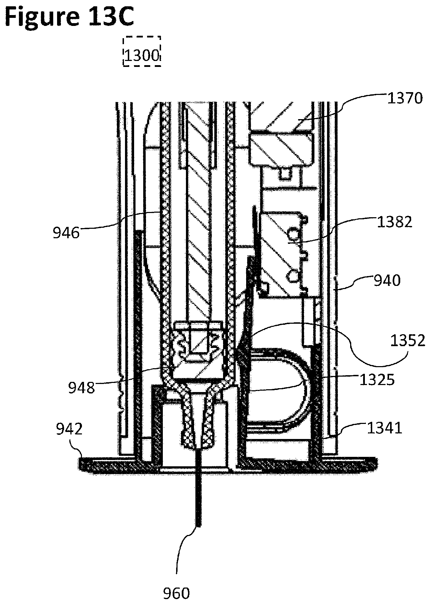

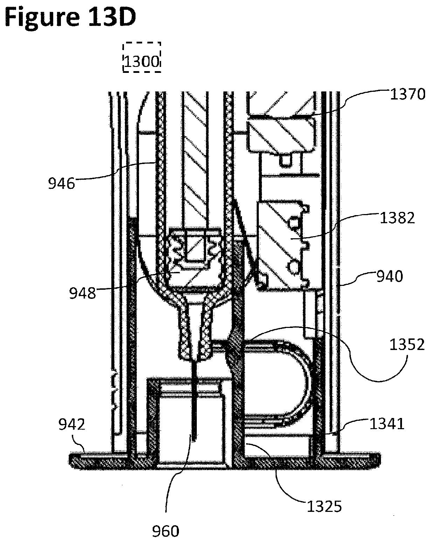

FIG 13C illustrates injector **1300,** immediately at the end of injection in accordance with an embodiment of the current invention. Optionally for as long as shield **1341** remains in a collapsed state and/or needle **960** remains in an extended state (protruding from the injector), extension **1325** continues to hold switch **1382** in an activated state and/or a motor continues to drive plunger **948.** Optionally, when plunger **948** reaches the end of syringe **946** a retraction mechanism (for example lock **758**) retracts syringe **946** and/or needle **960** to a retracted state (as illustrated for example in FIG. 13D).

FIG. 13D illustrates injector **1300** in a retracted state in accordance with an embodiment of the current invention. Optionally, when needle **960** and/or syringe **946** is retracted, element **1352** is released and/or extension **1325** moves away from switch **1382** and/or switch **1382** moves into a deactivated state and/or the motor is switched off.

## Claims

1. A drug injector (900) having a needle safeguard mechanism comprising:
a housing (440, 540, 940, 1040, 1140) including a shielded location;
a needle having a point (460, 560, 960) which protrudes from the housing (440, 540, 940, 1040, 1140);
a retractor (1058) mechanically coupled to the needle, the retractor (1058) retracting the point (460, 560, 960) to the shielded location; **characterized in that**
a sensor is operationally linked to the retractor (1058), the sensor sensing a resistance to discharging of a drug through the needle;
and that the retractor (1058) is responsive to the sensor to retract the point (460, 560, 960) to the shielded location in response to the resistance.

2. The drug injector of claim 1, wherein the retractor includes a support of the needle, and wherein the support reverts to a retracted state in response to the resistance.

3. The drug injector of claim 2, wherein the support includes an interference element (452a, b; 652; 752a, b; 852, 952, 1052), and wherein the resistance overcomes the interference element (452a, b; 652; 752a, b; 852, 952, 1052).

4. The drug injector of claim 3, wherein the interference element (452a, b; 652; 752a, b; 852, 952, 1052) includes an annular snap (952).

5. The drug injector of claim 2, wherein the support includes a telescoping assembly and wherein the telescoping assembly is held in an extended mode by an interference element (452a, b; 652; 752a, b; 852, 952, 1052).

6. The drug injector of claim 2, further comprising a driver (444, 544, 944, 1044) driving discharging of the drug,
wherein the driver (444, 544, 944, 1044) is supported by the support, and
wherein, in response to the resistance, the driver (444, 544, 944, 1044) produces a stress on the support and the stress reverts the support to the retracted state.

7. The drug injector of claim 6, further comprising a plunger (448, 548, 948),
wherein the driver (444, 544, 944, 1044) drives discharging of the drug by imparting a linear motion to the plunger (448, 548, 948), and
wherein the resistance is a resistance to the linear motion of the plunger (448, 548, 948).

8. The drug injector of claim 1, further comprising a lock (458, 658, 758) mechanically coupled to the needle (960), the lock (458, 658, 758) retaining the point in the protruding position,
wherein the retractor is operationally linked to the lock (458, 658, 758) to release the lock (458, 658, 758) in response to the resistance.

9. The drug injector of claim 8, wherein the sensor is further sensitive to a second condition, and wherein the lock (458, 658, 758) retains the point in the protruding position until the second condition is met.

10. The drug injector of claim 9, wherein the point is biased to the shielded location.

11. The drug injector of claim 8, wherein the lock (458, 658, 758) includes at least one element selected from the group consisting of an interference element (452a, b; 652; 752a, b; 852, 952, 1052), an annular snap (952), a rib snap, a flange and a groove.

12. The drug injector of claim 9, wherein the second condition is a volume of discharging of the drug passing a predetermined threshold.

13. The drug injector of claim 1, wherein the sensor senses a torque.

14. The drug injector of claim 1, wherein the retracting is in response to an increase or a decrease in the resistance.

15. The drug injector of claim 1, wherein the retracting is in response the resistance passing a threshold.

## Patentansprüche

1. Arzneimittelinjektor (900) mit einem Nadelschutzmechanismus, umfassend:
ein Gehäuse (440, 540, 940, 1040, 1140) mit einer abgeschirmten Position;
eine Nadel mit einer Spitze (460, 560, 960), die aus dem Gehäuse (440, 540, 940, 940, 1040, 1140) herausragt;
einen Retraktor (1058), der mit der Nadel mechanisch verbunden ist,
wobei der Retraktor (1058) die Spitze (460, 560, 960) in die abgeschirmte Position zurückzieht; **dadurch gekennzeichnet, dass**
ein Sensor mit dem Retraktor (1058) wirkverbunden ist, wobei der Sensor einen Widerstand gegen die Abgabe eines Medikaments durch die Nadel erfasst;
und dass der Retraktor (1058) auf den Sensor anspricht, um die Spitze (460, 560, 960, 960) in Reaktion auf den Widerstand in die abgeschirmte Position zurückzuziehen.

2. Arzneimittelinjektor nach Anspruch 1, wobei der Retraktor einen Nadelträger aufweist und wobei der Träger in Reaktion auf den Widerstand in einen zurückgezogenen Zustand zurückkehrt.

3. Arzneimittelinjektor nach Anspruch 2, wobei der Träger ein Eingriffselement (452a, b; 652; 752a, b; 852, 952, 1052) aufweist und wobei der Widerstand das Eingriffselement (452a, b; 652; 752a, b; 852 952, 1052) überwindet.

4. Arzneimittelinjektor nach Anspruch 3, wobei das Eingriffselement (452a, b; 652; 752a, b; 852, 952, 1052) einen ringförmigen Schnapper (952) umfasst.

5. Arzneimittelinjektor nach Anspruch 2, wobei der Träger eine Teleskopanordnung umfasst und wobei die Teleskopanordnung durch ein Eingriffselement (452a, b; 652; 752a, b; 852, 952, 1052) in einem ausgefahrenen Modus gehalten wird.

6. Arzneimittelinjektor nach Anspruch 2, ferner umfassend einen Treiber (444, 544, 944, 1044), der die Abgabe des Arzneimittels vorantreibt,
wobei der Treiber (444, 544, 944, 1044) durch den Träger gestützt ist und wobei der Treiber (444, 544, 944, 1044) in Reaktion auf den Widerstand eine auf den Träger wirkende Spannung erzeugt und die Spannung den Träger in den eingefahrenen Zustand zurückkehren lässt.

7. Arzneimittelinjektor nach Anspruch 6, ferner umfassend einen Kolben (448, 548, 948), wobei der Treiber (444, 544, 944, 1044) die Abgabe des Arzneimittels vorantreibt, indem dem Kolben (448, 548, 948) eine lineare Bewegung aufgeprägt wird, und
wobei der Widerstand ein Widerstand gegen die lineare Bewegung des Kolbens (448, 548, 948) ist.

8. Arzneimittelinjektor nach Anspruch 1, ferner umfassend eine Verriegelung (458, 658, 758), die mit der Nadel (960) mechanisch verbunden ist, wobei die Verriegelung (458, 658, 758) die Spitze in der vorspringenden Position hält,
wobei der Retraktor mit der Verriegelung (458, 658, 758) wirkverbunden ist, um die Verriegelung (458, 658, 758) in Reaktion auf den Widerstand zu lösen.

9. Arzneimittelinjektor nach Anspruch 8, wobei der Sensor ferner gegenüber einer zweiten Bedingung empfindlich ist und wobei die Verriegelung (458, 658, 758) die Spitze in der vorspringenden Position hält, bis die zweite Bedingung erfüllt ist.

10. Arzneimittelinjektor nach Anspruch 9, wobei die Spitze in die abgeschirmte Position vorgespannt ist.

11. Arzneimittelinjektor nach Anspruch 8, wobei die Verriegelung (458, 658, 758) mindestens ein Element umfasst, das aus der Gruppe ausgewählt ist, die aus einem Eingriffselement (452a, b; 652; 752a, b; 852, 952, 1052), einem ringförmigen Schnapper (952), einem rippenförmigen Schnapper, einem Flansch und einer Nut besteht.

12. Arzneimittelinjektor nach Anspruch 9, wobei der zweite Bedingung ist, dass ein Abgabevolumen des Arzneimittels eine vorgegebene Schwelle überschreitet.

13. Arzneimittelinjektor nach Anspruch 1, wobei der Sensor ein Drehmoment erfasst.

14. Arzneimittelinjektor nach Anspruch 1, wobei das Zurückziehen in Reaktion auf eine Zunahme oder eine Abnahme des Widerstands erfolgt.

15. Arzneimittelinjektor nach Anspruch 1, wobei das Zurückziehen in Reaktion darauf erfolgt, dass der Widerstand eine Schwelle überschreitet.

## Revendications

1. Injecteur de médicament (900) ayant un mécanisme de protection d'aiguille comprenant :
un boîtier (440, 540, 940, 1040, 1140) comprenant un espace protégé;
une aiguille ayant une pointe (460, 560, 960) qui fait saillie du boîtier (440, 540, 940, 1040, 1140);
un rétracteur (1058) couplé mécaniquement à l'aiguille, le rétracteur (1058) rétractant la pointe (460, 560, 960) vers l'espace protégé;
**caractérisé en ce que**
un capteur est relié de manière opérationnelle au rétracteur (1058), le capteur détectant une résistance à la décharge d'un médicament à travers l'aiguille ;
et **en ce que** le rétracteur (1058) est sensible au capteur pour rétracter la pointe (460, 560, 960) dans l'espace protégé en réponse à la résistance.

2. Injecteur de médicament selon la revendication 1, dans lequel le rétracteur comprend un support de l'aiguille et dans lequel le support revient à un état rétracté en réponse à la résistance.

3. Injecteur de médicament selon la revendication 2, dans lequel le support comprend un élément d'interférence (452a, b; 652; 752a, b; 852, 952, 1052), et dans la résistance dépasse l'élément d'interférence (452a, b; 652; 752a, b; 852, 952, 1052).

4. Injecteur de médicament selon la revendication 3, dans lequel l'élément d'interférence (452a, b; 652; 752a, b; 852, 952, 1052) comprend un dispositif d'encliquetage annulaire (952).

5. Injecteur de médicament selon la revendication 2, dans lequel le support comprend un ensemble télescopique et dans lequel l'ensemble télescopique est maintenu dans un mode étendu par un élément d'interférence (452a, b; 652; 752a, b; 852, 952, 1052).

6. Injecteur de médicament selon la revendication 2, comprenant en outre un pousseur (444, 544, 944, 1044) faisant décharger le médicament, dans lequel le pousseur (444, 544, 944, 1044) est supporté par le support, et dans lequel, en réponse à la résistance, le pousseur (444, 544, 944, 1044) produit une contrainte sur le support et la contrainte ramène le support à l'état rétracté.

7. Injecteur de médicament selon la revendication 6, comprenant en outre un piston (448, 548, 948, 948), dans lequel le pousseur (444, 544, 944, 944, 1044) entraîne la décharge du médicament en donnant un mouvement linéaire au piston (448, 548, 948), et
dans laquelle la résistance est une résistance au mouvement linéaire du piston (448, 548, 948).

8. Injecteur de médicament selon la revendication 1, comprenant en outre une serrure (458, 658, 758) couplée mécaniquement à l'aiguille (960), la serrure (458, 658, 758) maintenant la pointe en position saillante,
le rétracteur étant relié de manière opérationnelle à la serrure (458, 658, 758) pour libérer la serrure (458, 658, 758) en réponse à la résistance.

9. Injecteur de médicament selon la revendication 8, dans lequel le capteur est en outre sensible à une deuxième condition, et dans lequel la serrure (458, 658, 758) maintient la pointe dans la position en saillie jusqu'à ce que la deuxième condition soit remplie.

10. Injecteur de médicament selon la revendication 9, dans lequel la pointe est sollicitée vers l'espace protégé.

11. Injecteur de médicament selon la revendication 8, dans lequel la serrure (458, 658, 758) comprend au moins un élément choisi parmi le groupe constitué par un élément d'interférence (452a, b; 652; 752a, b; 852, 952, 1052), un dispositif d'encliquetage annulaire (952), un dispositif d'encliquetage en forme de nervure, une bride et une gorge.

12. Injecteur de médicament selon la revendication 9, dans lequel la deuxième condition est un volume de décharge du médicament passant un seuil prédéterminé.

13. Injecteur de médicament selon la revendication 1, dans lequel le capteur détecte un couple.

14. Injecteur de médicament selon la revendication 1, dans lequel la rétraction a lieu en réponse à une augmentation ou une diminution de la résistance.

15. Injecteur de médicament selon la revendication 1, dans lequel la rétraction s'effectue en réponse à la résistance passant un seuil.
